# EUROPÄISCHE PATENTANMELDUNG

(11) **EP 4 644 865 A1**
(43) Veröffentlichungstag der Anmeldung: **05.11.2025**
(21) Anmeldenummer: 24174153.7
(22) Anmeldetag: 03.05.2024
(51) Int. Cl.: G01N 21/17, A61B 5/08, G01N 29/24, G01N 33/497

(54) **VORRICHTUNG UND VERFAHREN ZUR ERFASSUNG VON ZUMINDEST ZWEI SPURENGASEN IN EINER AUSATEMLUFT**

(71) Anmelder: Fraunhofer-Gesellschaft zur Förderung der angewandten Forschung e.V., 80686 München (DE); Klinikum der Universität München Anstalt des öffentlichen Rechts, 81377 München (DE)
(72) Erfinder: ANGSTER, Judit, 70569 Stuttgart (DE); RUCZ, Peter, 70569 Stuttgart (DE); MIKLOS, Andras, 70569 Stuttgart (DE); MEIDERT, Agnes, 81377 München (DE); SCHELLING, Gustav, 81377 München (DE)
(74) Vertreter: Friese Goeden Patentanwälte PartGmbB

(57) **Zusammenfassung**

Beschrieben wird eine Vorrichtung (9) zur Erfassung von zumindest zwei Spurengasen in einer Ausatemluft, enthaltend eine erste photoakustische Messeinrichtung (1) mit einem ersten akustischen Resonator (10), zumindest einem ersten Mikrofon (11) und zumindest einer ersten Lichtquelle (12), eine Einrichtung (3) zur Erfassung einer Resonanz-frequenz einer Resonatormode des ersten akustischen Resonators (10), eine Einrichtung (4) zur Modulation der ersten Lichtquelle (12) mit der Resonanzfrequenz der Resonatormode, wobei die Vorrichtung (9) weiterhin eine zweite photoakustische Messeinrichtung (2) enthält, mit einem zweiten akustischen Resonator (20), zumindest einem zweiten Mikrofon (21) und zumindest einer zweiten Lichtquelle (22), wobei die Einrichtung (4) zur Modulation der ersten Lichtquelle (12) dazu eingerichtet ist, auch die zweite Lichtquelle (22) mit der Resonanzfrequenz der Resonatormode des ersten akustischen Resonators (10) zu modulieren oder eine weitere Einrichtung (3) zur Erfassung einer Resonanzfrequenz einer Resonatormode des zweiten akustischen Resonators (20) und eine weitere Einrichtung (4) zur Modulation der zweiten Lichtquelle (12) mit der Resonanzfrequenz der Resonatormode des zweiten akustischen Resonators (20) vorhanden sind. Weiterhin wird ein Verfahren zur Bestimmung von zumindest zwei Spurengasen in einer Ausatemluft beschrieben.

## Beschreibung

Die Erfindung betrifft eine Vorrichtung und ein Verfahren zur Erfassung von zumindest zwei Spurengasen in einer Ausatemluft, enthaltend eine erste photoakustische Messeinrichtung mit einem ersten akustischen Resonator, zumindest einem ersten Mikrofon und zumindest einer ersten Lichtquelle, eine Einrichtung zur Erfassung einer Resonanzfrequenz einer Resonatormode des ersten akustischen Resonators sowie einer Einrichtung zur Modulation der ersten Lichtquelle mit der Resonanzfrequenz der Resonatormode. Weiterhin betrifft die Erfindung ein Verfahren zur Erfassung von zumindest zwei Spurengasen in einer Ausatmenluft. Vorrichtungen und Verfahren dieser Art können zur medizinischen Diagnostik verwendet werden, beispielsweise zur Bestimmung des Wirkspiegels von Narkosemitteln.

Aus T. Laurila, T. Sorvajärvi, J. Saarela, J. Toivonen, D. W. Wheeler, L. Ciaffoni, G. A. D. Ritchie, and C. F. Kaminski: "Optical Detection of the Anesthetic Agent Propofol in the Gas Phase", Analytical Chemistry 2011 83 (10), 3963-3967 ist bekannt, das Anästhetikum Propofol mittels einer optoakustischen Messung in der Ausatemluft eines Patienten während einer total-intravenösen Anästhesie (TIVA) zu bestimmen. Hierbei wird das zu untersuchende Gas in einen akustischen Resonator eingebracht und durch Licht einer vorgebbaren Wellenlänge angeregt. Die Wellenlänge ist dabei so ausgewählt, dass diese einen optischen Übergang im Zielmolekül resonant anregen kann. Der angeregte Zustand des Zielmoleküls zerfällt nichtstrahlend, wodurch eine Temperatur- und Druckschwankung im Gas auftritt. Sofern die Lichtquelle mit einer Resonanzfrequenz einer Resonatormode des akustischen Resonators betrieben wird, kann das photoakustische Signal im akustischen Resonator verstärkt und mittels eines Mikrofons nachgewiesen werden.

Diese bekannte Vorrichtung weist den Nachteil auf, dass unterschiedliche Spurengase überlappende Absorptionsbanden aufweisen können, sodass die Genauigkeit und/oder die Nachweisgrenze für ein vorgebbares Zielmolekül reduziert sind. Ausgehend vom Stand der Technik könnte eine mögliche Aufgabe der Erfindung darin bestehen, eine Vorrichtung und ein Verfahren zur Erfassung von Spurengasen mit höherer Genauigkeit anzugeben.

Die nachfolgende Beschreibung beschreibt eine Vorrichtung gemäß Anspruch 1 und ein Verfahren nach Anspruch 11. Gegebenenfalls vorteilhafte Weiterbildungen finden sich in den Unteransprüchen.

Gemäß einem Aspekt wird eine Vorrichtung zur Erfassung von zumindest zwei Spurengasen offenbart. Die Spurengase können in einer Ausatemluft eines Lebewesens vorhanden sein, insbesondere eines Menschen. In einigen Ausführungsformen der Erfindung kann die Vorrichtung dazu eingerichtet sein, die Ausatemluft einer Beatmungsmaschine beziehungsweise einem Narkosekreisteil zu entnehmen.

Gemäß einem Aspekt wird ein Verfahren zur Erfassung von zumindest zwei Spurengasen offenbart. Die Spurengase können in einer Ausatemluft eines Lebewesens, insbesondere eines Menschen vorhanden sein. In einigen Ausführungsformen der Erfindung kann die Ausatemluft einer Beatmungsmaschine beziehungsweise einem Narkosekreisteil entnommen werden. In einigen Ausführungsformen kann das Verfahren gemäß dem zweiten Aspekt mit einer Vorrichtung gemäß dem ersten Aspekt der vorliegenden Beschreibung durchgeführt werden.

Die Vorrichtung gemäß dem einen Aspekt der vorliegenden Beschreibung kann eine erste photoakustische Messeinrichtung aufweisen. Die erste photoakustische Messeinrichtung kann einen ersten akustischen Resonator, zumindest ein erstes Mikrofon und zumindest eine erste Lichtquelle enthalten. Der erste akustische Resonator kann in einigen Ausführungsformen der Erfindung ein Hohlkörper sein, in welchen eine zu analysierende Gasprobe eingebracht wird.

Die zu analysierende Gasprobe kann die Ausatemluft eines Lebewesens sein oder diese enthalten. Unter einer "Ausatemluft" wird für die Zwecke der vorliegenden Beschreibung ein Gas verstanden, welches die Lunge des Lebewesens verlässt. Ungeachtet der Bezeichnung als "Luft" kann diese auch ein Gas sein oder enthalten, welches in komprimierter oder tiefkalter Form bereitgestellt worden ist, beispielsweise ein Narkosegas oder medizinischer Sauerstoff. Ungeachtet der Bezeichnung als "Luft" kann die molekulare Zusammensetzung gegenüber der natürlichen Umgebungsluft modfiziert sein, beispielsweise kann der Sauerstoffgehalt verringert und der Kohlendioxidgehalt erhöht sein. Daneben kann die Ausatemluft Spurengase enthalten, beispielsweise Metabolite des Lebewesens.

Der erste akustische Resonator kann aus einem Metall oder einer Legierung oder einem Kunststoff oder Glas oder Keramik gefertigt sein. Der erste akustische Resonator kann eine elliptische, runde oder polygonale Grundform aufweisen. Der erste akustische Resonator kann zumindest eine Eigenmode bzw. Resonanzfrequenz aufweisen, welche von den geometrischen Abmessungen und der Form des ersten Resonators und der Schallgeschwindigkeit des im ersten Resonator enthaltenen Gases abhängt. Die Schallgeschwindigkeit kann durch die atomare beziehungsweise molekulare Zusammensetzung des enthaltenen Gases und dessen Temperatur beeinflusst sein. Der erste akustische Resonator kann dazu eingerichtet und bestimmt sein, Schallemissionen innerhalb des enthaltenen Gases zu verstärken. Hierzu kann vorgesehen sein, die Schallemissionen mit der Frequenz zumindest einer Eigenmode des ersten akustischen Resonators anzuregen. Die Frequenz zumindest einer Eigenmode wird im weiteren Verlauf der Beschreibung auch als Resonanzfrequenz einer Resonatormode des ersten akustischen Resonators bezeichnet.

Zum Nachweis von Spurengasen können die in der Ausatemluft enthaltenen Moleküle des Spurengases optisch angeregt werden. Unter einer optischen Anregung wird für die Zwecke der vorliegenden Beschreibung die resonante Absorption von Licht verstanden, d.h. die Photonenenergie des Lichtes entspricht der Energie eines elektronischen oder vibronischen Übergangs im Molekül. Hierzu wird Licht aus zumindest einer ersten Lichtquelle in das im ersten akustischen Resonator enthaltene Gas eingestrahlt. Durch die Absorption des Lichtes werden die nachzuweisenden Zielmoleküle in einen energetisch höheren Zustand angeregt. Die nachzuweisenden Zielmoleküle können diese Anregungsenergie durch nicht-strahlende Übergänge abgeben, wodurch eine lokale Temperaturerhöhung hervorgerufen werden kann. Diese kann zu einer lokalen Erhöhung des Druckes führen. Sofern die erste Lichtquelle mit der Resonanzfrequenz einer Resonatormode des ersten akustischen Resonators moduliert wird, wird ein akustisches Signal mit der Resonanzfrequenz erzeugt und im ersten akustischen Resonator verstärkt. Die Intensität dieses akustischen Signals ist ein Maß für die Konzentration des Zielmoleküls. Das akustische Signal kann mit zumindest einem ersten Mikrofon erfasst werden. Unter einem nichtstrahlenden Übergang wird für die Zwecke der vorliegenden Beschreibung die Änderung des Energieniveaus zumindest eines Elektrons in einem Molekül verstanden, bei welchem kein Photon emittiert wird. Ein nicht-strahlender Übergang kann in einigen Ausführungsformen ausgewählt sein aus dem Auger-Effekt, einer Stoßionisationen oder einem vibronische Übergang, bei welchem ein Molekül bei der Wechselwirkung mit einem Photon seine Schwingungs- oder Rotationsfrequenz ändert.

Das zumindest eine erste Mikrofon kann in einigen Ausführungsformen der Erfindung ein MEMS-Mikrofon und/oder ein Kondensatormikrofon sein. Ein MEMS-Mikrofon kann in einigen Ausführungsformen der Erfindung einen monolithisch integrierten Verstärker aufweisen. Hierdurch kann sich ein kompakter und robuster Aufbau bei gleichzeitig hoher Empfindlichkeit und geringer Störungsanfälligkeit ergeben.

Wie vorstehend ausgeführt, ändert sich die Resonanzfrequenz der Eigenmoden des ersten akustischen Resonators in Abhängigkeit der Schallgeschwindigkeit und damit in Abhängigkeit der molekularen Zusammensetzung und der Temperatur. Zur Erhöhung der Genauigkeit kann daher eine Einrichtung zur Erfassung einer Resonanzfrequenz einer Resonatormode des ersten akustischen Resonators vorhanden sein. Diese kann die Resonanzfrequenz fortlaufend oder in vorgebbaren Zeitintervallen erfassen und damit die Einrichtung zur Modulation der ersten Lichtquelle steuern bzw. regeln. Hierdurch kann die Genauigkeit erhöht sein, da die Modulation der Lichtquelle stets so erfolgt, dass die photoakustischen Signale der Zielmoleküle im ersten akustischen Resonator verstärkt werden.

In einigen Ausführungsformen der Erfindung kann die Vorrichtung weiterhin eine zweite photoakustische Messeinrichtung enthalten, welche einen zweiten akustischen Resonator, zumindest ein zweites Mikrofon und zumindest eine zweite Lichtquelle enthält. Der zweite akustische Resonator kann in einigen Ausführungsformen der Erfindung ein Hohlkörper sein, in welchen eine zu analysierende Gasprobe eingebracht wird.

Der zweite akustische Resonator kann aus einem Metall oder einer Legierung oder einem Kunststoff oder Glas oder Keramik gefertigt sein. Der zweite akustische Resonator kann eine elliptische, runde oder polygonale Grundform aufweisen. Der zweite akustische Resonator kann zumindest eine Eigenmode bzw. Resonanzfrequenz aufweisen, welche von den geometrischen Abmessungen und der Form des Resonators und der Schallgeschwindigkeit des im Resonator enthaltenen Gases abhängt. In einigen Ausführungsformen der Erfindung können die Form und/oder die Maße des ersten akustischen Resonators mit der Form und/oder den Maßen des zweiten akustischen Resonators übereinstimmen, so dass die Eigenmoden bzw. Resonanzfrequenzen beider Resonatoren nahezu identisch sind. Beispielsweise kann die Abweichung geringer sein als etwa 5 Hz oder geringer als etwa 2 Hz oder geringer als etwa 1 Hz. Der zweite akustische Resonator kann dazu eingerichtet und bestimmt sein, Schallemissionen innerhalb des Gases zu verstärken. Hierzu kann vorgesehen sein, die Schallemissionen mit der Frequenz zumindest einer Eigenmode des zweiten akustischen Resonators anzuregen. Die Frequenz zumindest einer Eigenmode wird im weiteren Verlauf der Beschreibung auch als Resonanzfrequenz einer Resonatormode des zweiten akustischen Resonators bezeichnet.

Zum Nachweis von Spurengasen können die in der Ausatemluft enthaltenen Moleküle des Spurengases optisch angeregt werden. Unter einer optischen Anregung wird für die Zwecke der vorliegenden Beschreibung die resonante Absorption von Licht verstanden, d.h. die Photonenenergie des Lichtes entspricht der Energie eines elektronischen oder vibronischen Übergangs im Molekül. Hierzu wird Licht aus zumindest einer zweiten Lichtquelle in das im zweiten akustischen Resonator enthaltene Gas eingestrahlt. Durch die Absorption des Lichtes werden die nachzuweisenden Zielmoleküle in einen energetisch höheren Zustand angeregt. Die nachzuweisenden Zielmoleküle können diese Anregungsenergie durch nicht-strahlende Übergänge abgeben, wodurch eine Temperaturerhöhung hervorgerufen wird. Sofern die zweite Lichtquelle mit der Resonanzfrequenz einer Resonatormode des zweiten akustischen Resonators moduliert wird, wird ein akustisches Signal mit der Resonanzfrequenz erzeugt und im zweiten akustischen Resonator verstärkt. Die Intensität dieses akustischen Signals ist ein Maß für die Konzentration des Zielmoleküls. Das akustische Signal kann mit zumindest einem zweiten Mikrofon erfasst werden.

Das zumindest eine zweite Mikrofon kann in einigen Ausführungsformen der Erfindung ein MEMS-Mikrofon und/oder ein Kondensatormikrofon sein. Ein MEMS-Mikrofon kann in einigen Ausführungsformen der Erfindung einen monolithisch integrierten Verstärker aufweisen. Hierdurch kann sich ein kompakter und robuster Aufbau bei gleichzeitig hoher Empfindlichkeit und geringer Störungsanfälligkeit ergeben.

Wie vorstehend ausgeführt, ändert sich die Resonanzfrequenz der Eigenmoden des zweiten akustischen Resonators in Abhängigkeit der Schallgeschwindigkeit und damit in Abhängigkeit der molekularen Zusammensetzung und der Temperatur. Zur Erhöhung der Genauigkeit kann daher in einigen Ausführungsformen der Erfindung eine Einrichtung zur Erfassung einer Resonanzfrequenz einer Resonatormode des zweiten akustischen Resonators vorhanden sein. Diese kann die Resonanzfrequenz fortlaufend oder in vorgebbaren Zeitintervallen erfassen und damit die Einrichtung zur Modulation der zweiten Lichtquelle steuern bzw. regeln. Hierdurch kann die Genauigkeit erhöht sein, da die Modulation der Lichtquelle stets so erfolgt, dass die photoakustischen Signale der Zielmoleküle im zweiten akustischen Resonator verstärkt werden. In diesem Fall kann die Genauigkeit der Messung erhöht sein, weil Unterschiede in der Resonanzfrequenz beider Resonatoren, welche durch abweichende geometrische Abmessungen und/oder abweichende Temperaturen und/oder eine abweichende Zusammensetzung des im jeweiligen Resonator enthaltenen Gases hervorgerufen werden, Berücksichtigung finden.

In einigen Ausführungsformen der Erfindung kann die Einrichtung zur Modulation der ersten Lichtquelle dazu eingerichtet sein, auch die zweite Lichtquelle mit der Resonanzfrequenz der Resonatormode des ersten akustischen Resonators zu modulieren. Dies ist insbesondere dann hilfreich, wenn die geometrischen Abmessungen und damit die Resonanzfrequenzen der Resonatormoden des ersten und zweiten akustischen Resonators identisch sind. Hierdurch kann zusätzlicher Aufwand zur Ermittlung der Resonanzfrequenz der Resonatormode des zweiten akustischen Resonators eingespart werden.

In einigen Ausführungsformen der Erfindung kann die Einrichtung zur Modulation die erste und/oder zweite Lichtquelle durch direkte Modulation des Versorgungsstroms bzw. der Versorgungsspannung modulieren. Somit kann die Einrichtung zur Modulation die erste und/oder zweite Lichtquelle auch mit elektrischer Energie versorgen. In anderen Ausführungsformen der Erfindung kann die Einrichtung zur Modulation einen Modulator ansteuern, welcher den Lichtstrahl der ersten und/oder zweiten Lichtquelle unterbricht bzw. freigibt. Der Modulator kann ausgewählt sein aus einem mechanischen Shutter oder einer Kerr-Zelle oder einer Pockels-Zelle oder einem LCD-Element. In wiederum anderen Ausführungsformen der Erfindung kann die Einrichtung zur Modulation sowohl eine direkte Modulation des Versorgungsstroms bzw. der Versorgungsspannung als auch die Ansteuerung eines Modulators bewirken.

In einigen Ausführungsformen der Erfindung kann der erste und/oder zweite akustische Resonator an zumindest einen Hohlkörper angrenzen bzw. mit zumindest einem Hohlkörper verbunden sein. In einigen Ausführungsformen können jeweils zwei Hohlkörper an gegenüberliegenden Enden des ersten und zweiten akustische Resonator angrenzen. Die Hohlkörper können jeweils ein Puffervolumen zur Verfügung stellen. Dieses kann in einzelnen Ausführungsformen der Erfindung folgende Wirkungen haben, wobei nicht alle Vorteile in allen Ausführungsformen gleichermaßen realisiert sein müssen:
- Sie sorgen dafür, dass die offenen Enden des Resonators den photoakustisch erzeugten Schalldruck reflektieren. In einigen Ausführungsformen können mehr als 90 % des photoakustisch erzeugten Schalldrucks reflektiert werden.
- In den Hohlkörpern oder in einem Hohlkörper können die Zu- und Abfuhrleitungen münden. Hierdurch wird eine Störung des photoakustischen Signals durch Strömungsgeräusche verhindert oder reduziert.
- Der oder die Hohlkörper können Teil eines freilaufenden akustischen Oszillators sein, welcher die Erfassung der Frequenz zumindest einer Eigenmode des akustischen Resonators ermöglicht, so dass die Modulationsfrequenz der Lichtquelle bestimmt werden kann.

Die erste und/oder zweite Lichtquelle kann in einigen Ausführungsformen der Erfindung ausgewählt sein aus einer Leuchtdiode und/oder einer Superlumineszenzdiode und/oder einem Halbleiterlaser. In einigen Ausführungsformen der Erfindung können die erste und die zweite Lichtquelle unterschiedliche Wellenlängen bzw. Frequenzen aufweisen. Dies ermöglicht es, in der ersten photoakustischen Messeinrichtung ein erstes Spurengas nachzuweisen und in der zweiten photoakustischen Messeinrichtung ein zweites Spurengas nachzuweisen. Dabei kann das erste Spurengas vom zweiten Spurengas verschieden sein. In einigen Ausführungsformen der Erfindung kann in der ersten photoakustischen Messeinrichtung die Summe beider Spurengase nachgewiesen werden, wohingegen in der zweiten photoakustischen Messeinrichtung nur eines der beiden Spurengase nachgewiesen wird. Damit ergibt sich die Konzentration des anderen Spurengases aus der Differenz der beiden Messwerte. Im Ergebnis kann somit die Konzentration von zwei Spurengasen in der Ausatemluft zuverlässig bestimmt werden.

Die erste und/oder zweite Lichtquelle kann in einigen Ausführungsformen der Erfindung mit einer Optik versehen sein, welche dazu eingerichtet und bestimmt sein kann, das von der Lichtquelle ausgehende Licht zu sammeln und/oder zu kollimieren und/oder in ein Zielvolumen innerhalb des ersten bzw. zweiten akustischen Resonators zu fokussieren. Hierdurch kann die Signalintensität und/oder das Signal/Rauschverhältnis erhöht sein. Die Optik kann in einigen Ausführungsformen eine Linse enthalten oder daraus bestehen. In anderen Ausführungsformen der Erfindung kann die Optik eine Mehrzahl von Linsen bzw. ein Linsensystem enthalten oder daraus bestehen. In einigen Ausführungsformen der Erfindung kann die Optik einen Kollimator und/oder eine Blende enthalten oder daraus bestehen.

In einigen Ausführungsformen der Erfindung können der erste akustische Resonator und der zweite akustische Resonator in Strömungsrichtung der Ausatemluft hintereinander angeordnet sein. Die Ausatemluft durchströmt somit zunächst den ersten akustischen Resonator und in der Folge den zweiten akustischen Resonator. Die photoakustische Messung wird somit an einer vorgebbaren Gasmenge sequenziell mit beiden photoakustischen Messeinrichtungen vorgenommen, um auf diese Weise unterschiedliche Spurengase in einer einzigen Gasprobe zu bestimmen.

In einigen Ausführungsformen der Erfindung können der erste akustische Resonator und der zweite akustische Resonator eine gemeinsame Trennwand aufweisen, welche eine Bohrung aufweist, welche ein Überströmen des zugeführten Gases vom ersten Resonator in den zweiten Resonator ermöglicht. Die Bohrung kann in einigen Ausführungsformen der Erfindung einen Durchmesser von etwa 0,1 mm bis etwa 1 mm oder von etwa 0,5 mm bis etwa 2 mm aufweisen. Hierdurch kann ein Überströmen des Gases ermöglicht werden, ohne eine akustische Kopplung der beiden Resonatoren zu bewirken. Hierdurch kann das Übersprechen des photoakustischen Signals eines Resonators in das Mikrofon des anderen Resonators zumindest reduziert sein.

In einigen Ausführungsformen der Erfindung kann die erste Lichtquelle dazu eingerichtet sein, Licht mit einer Wellenlänge von etwa 270 nm bis etwa 300 nm zu emittieren. In anderen Ausführungsformen der Erfindung kann die erste Lichtquelle dazu eingerichtet sein, Licht mit einer Wellenlänge von etwa 270 nm bis etwa 280 nm zu emittieren. Hierdurch kann die erste photoakustische Messeinrichtung dazu eingerichtet sein, die Spurengase Aceton und Propofol zu erfassen.

In einigen Ausführungsformen der Erfindung kann die zweite Lichtquelle dazu eingerichtet sein, Licht mit einer Wellenlänge von etwa 290 nm bis etwa 310 nm zu emittieren. Hierdurch kann die zweite photoakustische Messeinrichtung dazu eingerichtet sein, das Spurengas Aceton zu erfassen.

In einigen Ausführungsformen der Erfindung kann die erste photoakustische Messeinrichtung eine Mehrzahl erster Lichtquellen aufweisen. Die einzelnen Lichtquellen einer Mehrzahl erster Lichtquellen können dazu eingerichtet und bestimmt sein, jeweils eine unterschiedliche Wellenlänge zu emittieren. In einigen Ausführungsformen der Erfindung kann die zweite photoakustische Messeinrichtung eine Mehrzahl zweiter Lichtquellen aufweisen. Die einzelnen Lichtquellen einer Mehrzahl zweiter Lichtquellen können dazu eingerichtet und bestimmt sein, Licht unterschiedlicher Wellenlängen zu emittieren. Hierdurch können in jeder photoakustischen Messeinrichtung unterschiedliche Spurengase nachgewiesen werden oder die Selektivität kann erhöht sein, indem mehrere Absorptionslinien eines Zielmoleküls durch unterschiedliche Lichtquellen angeregt werden.

In einigen Ausführungsformen der Erfindung können die ersten Lichtquellen der ersten photoakustischen Messeinrichtung dazu eingerichtet sein, Licht mit 275 nm und 295 nm zu emittieren. Diese Wellenlängen werden von Propofol und Aceton absorbiert und können somit in Aceton- und Propofolhaltigen Gasen zur Ausbildubng eines photoakustischen Signals führen.

In einigen Ausführungsformen der Erfindung können die zweiten Lichtquellen der zweiten photoakustischen Messeinrichtung dazu eingerichtet sein, Licht mit 295 nm und 308 nm zu emittieren. Diese Wellenlängen werden von Aceton absorbiert und können somit in Aceton-haltigen Gasen zur Ausbildung eines photoakustischen Signals führen.

In einigen Ausführungsformen der Erfindung kann die Vorrichtung weiterhin zumindest eine Förderpumpe enthalten, welche dazu eingerichtet ist, die Ausatemluft bzw. ein anderes zu analysierendes Gas über eine Zufuhrleitung durch den ersten und zweiten akustischen Resonator zu fördern. Hierdurch kann ein kontinuierlicher Gasaustausch ermöglicht werden, welcher eine kontinuierliche Messung der Spurengase ermöglicht.

In einigen Ausführungsformen der Erfindung kann in der Abfuhrleitung eine Druckmesseinrichtung und ein Drosselventil vorhanden sein. In anderen Ausführungsformen der Erfindung kann in der Zufuhrleitung eine Druckmesseinrichtung und ein Drosselventil vorhanden sein. Die Druckmesseinrichtung kann dazu eingerichtet und bestimmt sein, einen Druckverlust über das Drosselventil zu erfassen. Hierdurch kann aus dem Druckverlust der Durchfluss durch die Zufuhrleitung und die photoakustischen Messeinrichtungen bestimmt werden. Die Zufuhrleitung ist dabei in Strömungsrichtung vor den photoakustischen Messeinrichtungen angeordnet. Die Abfuhrleitung ist in Strömungsrichtung hinter den photoakustischen Messeinrichtungen angeordnet.

In einigen Ausführungsformen der Erfindung enthält die Vorrichtung weiterhin zumindest eine Heizeinrichtung, mit welcher der erste akustische Resonator und/oder der zweite akustische Resonator und/oder die Zufuhrleitung beheizt werden können. Dies vermindert die Adsorption der Spurengase an den inneren Oberflächen der Zufuhrleitung und/oder der akustischen Resonatoren, sodass die Messgenauigkeit und/oder das Ansprechverhalten der Vorrichtung erhöht sein kann. In einigen Ausführungsformen der Erfindung kann die Heizeinrichtung dazu eingerichtet bzw. bestimmt sein, zumindest eine Teilfläche eines akustischen Resonators und/oder der Zufuhrleitung auf eine Temperatur von etwa 80° bis etwa 180° zu bringen. In anderen Ausführungsformen der Erfindung kann die Heizeinrichtung dazu bestimmt sein, zumindest eine Teilfläche eines akustischen Resonators und/oder eine Teilfläche der Zufuhrleitung auf eine Temperatur von etwa 100° bis etwa 120° zu bringen. Dies vermeidet zuverlässig die unerwünschte Adsorption der Spurengase an den inneren Oberflächen der Vorrichtung.

In einigen Ausführungsformen der Erfindung kann zumindest eine Teilfläche der Innenflächen der ersten akustischen Resonators und/oder zumindest eine Teilfläche der Innenflächen des zweiten akustischen Resonators und/oder zumindest eine Teilfläche der Innenflächen der Zufuhrleitung mit einer Beschichtung versehen sein. Eine solche Beschichtung kann alternativ oder zusätzlich zur Heizeinrichtung die Adsorption der nachzuweisenden Spurengase verhindern oder reduzieren.

In einigen Ausführungsformen der Erfindung kann eine solche Beschichtung Silizium und/oder Siliziumoxid enthalten oder daraus bestehen. Solche Beschichtungen sind einfach herstellbar und bilden nahezu inerte Oberflächen für eine Vielzahl von Spurengasen.

In einigen Ausführungsformen der Erfindung kann zwischen dem ersten Mikrofon und dem ersten akustischen Resonator ein erstes Verbindungsrohr angeordnet sein. In einigen Ausführungsformen kann zwischen dem zweiten Mikrofon und dem zweiten akustischen Resonator ein zweites Verbindungsrohr angeordnet sein. Das Verbindungsrohr bzw. die Verbindungsrohre kann in einigen Ausführungsformen der Erfindung Glas oder Kunststoff oder Keramik enthalten oder daraus bestehen. In einigen Ausführungsformen der Erfindung kann das Verbindungsrohr Polytetrafluorethylen enthalten oder daraus bestehen. Ein Verbindungsrohr kann einen Innendurchmesser von etwa 1 mm bis etwa 3 mm oder von etwa 1,5 mm aufweisen. Ein solches Verbindungsrohr kann die Wirkung haben, dass das erste bzw. zweite Mikrofon auf einer niedrigeren Temperatur gehalten werden kann als der erste bzw.zweite akustische Resonator. Hierdurch kann eine Beschädigung des ersten und/oder zweiten Mikrofons durch Übertemperaturen verhindert oder verzögert werden.

Nachstehend soll die Erfindung anhand von Figuren ohne Beschränkung des allgemeinen Erfindungsgedankens näher erläutert werden. Dabei zeigt
Figur 1 den allgemeinen Aufbau der Vorrichtung.
Figur 2 zeigt die photoakustische Messeinrichtung als Blockschaltbild.
Figur 3 zeigt einen vergößerten Ausschnitt as Fig. 2.
Figur 4 zeigt eine Ausführungsform eines photoakustischen Resonators im Schnitt.
Figur 5 zeigt die Modulationsfrequenz der ersten Lichtquelle und das photoakustische Signal des ersten Mikrofons in Abhängigkeit der Gaszusammensetzung.
Figur 6 zeigt ein Ablaufdiagramm des vorgeschlagenen Messverfahrens.

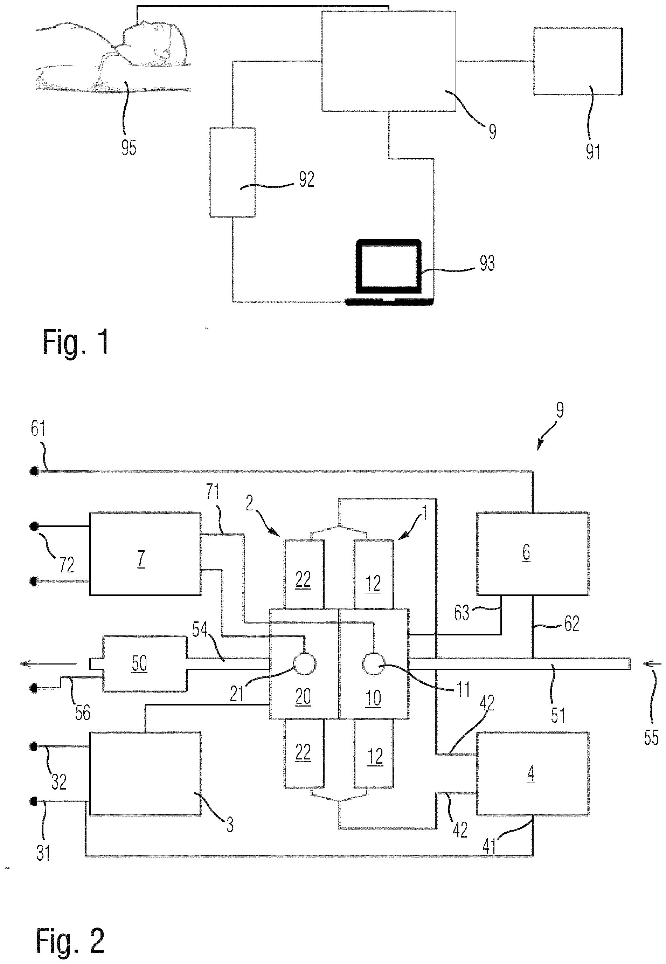

Figur 1 zeigt den allgemeinen Aufbau der hier beschriebenen Messeinrichtung. Die Messeinrichtung umfasst die anhand von Figur 2 näher erläuterte Vorrichtung 9 zur Erfassung von zumindest zwei Spurengasen in einer Ausatemluft. In einigen Ausführungsformen der Erfindung kann die Ausatemluft von einem Lebewesen stammen, insbesondere von einem beatmeten menschlichen Patient. In diesem Fall kann die Ausatemluft am Ausgang einer Beatmungsmaschine oder eines Narkosekreisteils entnommen und der Vorrichtung 9 zugeführt werden.

Weiterhin enthält die Messeinrichtung eine elektrische Stromversorgung 91, welche die Vorrichtung 9 mit elektrischer Energie versorgt. Die von der Vorrichtung 9 erzeugten Messwerte können mittels eines A/D-Wandlers 92 digitalisiert und einer elektronischen Datenverarbeitungsanlage 93 zugeführt werden. Die elektronische Datenverarbeitungsanlage 93 kann die Messwerte speichern oder visualisieren oder zur Steuerung bzw. Regelung weiterer Geräte bereitstellen. Darüber hinaus kann die elektronische Datenverarbeitungsanlage 93 Steuersignale erzeugen, welche die Vorrichtung 9 zur Erfassung von Spurengasen kontrolliert und in bestimmten Zeitabständen bestimmte Messungen durchführt.

Anhand der Figur 2 wird die Vorrichtung 9 zur Erfassung von zumindest zwei Spurengasen in einer Ausatemluft 55 näher erläutert. Die Vorrichtung 9 enthält zumindest eine erste photoakustische Messeinrichtung 1 und zumindest eine zweite photoakustische Messeinrichtung 2. Die erste photoakustische Messeinrichtung 1 weist einen ersten akustischen Resonator 10 auf, zumindest ein erstes Mikrofon 11 und zumindest eine erste Lichtquelle 12. Im dargestellten Ausführungsbeispiel sind zwei erste Lichtquellen 12 dargestellt, welche Licht unterschiedlicher Wellenlängen emittieren.

Die zweite photoakustische Messeinrichtung 2 weist einen zweiten akustischen Resonator 20 auf. Schallsignale im zweiten akustischen Resonator können mit zumindest einem zweiten Mikrofon 21 erfasst werden. Darüber hinaus enthält auch die zweite photoakustische Messeinrichtung zumindest eine zweite Lichtquelle 22. Im dargestellten Ausführungsbeispiel sind zwei zweite Lichtquellen 22 vorhanden, welche Licht unterschiedlicher Wellenlänge emittieren.

Die akustischen Resonatoren 10, 20 enthalten im Wesentlichen einen Hohlraum, welcher von der zu analysierenden Ausatemluft 55 durchströmt wird. Im dargestellten Ausführungsbeispiel sind der erste und zweite akustische Resonator 10, 20 in Strömungsrichtung der Ausatemluft 55 hintereinander angeordnet. Somit werden der erste akustische Resonator 10 und der zweite akustische Resonator 20 von der Ausatemluft 55 sequenziell durchströmt. In einigen Ausführungsformen der Erfindung können der erste und zweite akustische Resonator gleiche geometrische Abmessungen aufweisen, sodass diese gleiche Eigenmoden und gleiche Resonanzfrequenzen aufweisen. Hierdurch kann die Modulation der ersten und zweiten Lichtquelle vereinfacht sein, weil diese mit gleicher Frequenz moduliert werden können.

Die Strömung der Ausatemluft 55 durch die Zufuhrleitung 51 und die ersten und zweiten akustischen Resonatoren 10 und 20 wird durch eine Pumpe 50 aufrechterhalten. Die Pumpe 50 kann eine Mikropumpe sein. Die Pumpe 50 kann über eine Steuerleitung 56 ein- und ausgeschaltet werden, um auf diese Weise die Strömung der Ausatemluft 55 an die jeweilige Messung anzupassen. Die Strömung kann zwischen etwa 30 cm³/sec und etwa 150 cm³/sec oder zwischen etwa 50 cm³/sec und etwa 100 cm³/sec betragen.

Die Zufuhrleitung 51 sowie die inneren Flächen der akustischen Resonatoren 10 und 20 können mit einer Beschichtung versehen sein, welche die Adhäsion der nachzuweisenden Spurengase reduziert oder verhindert. Eine solche Beschichtung kann Silizium und/oder Siliziumoxid enthalten oder daraus bestehen. Darüber hinaus kann die Adsorption der Spurengase reduziert werden, wenn die Zufuhrleitung 51 und/oder der erste akustische Resonator 10 und/oder der zweite akustische Resonator 20 auf eine erhöhte Temperatur von beispielsweise etwa 80° bis etwa 180° oder etwa 100° bis etwa 120° gebracht werden. Hierzu kann eine Heizeinrichtung 6 zur Verfügung stehen, welche beispielsweise über eine elektrische Widerstandsheizung eine erhöhte Temperatur ermöglicht. Darüber hinaus kann die Heizeinrichtung 6 eine Steuer- oder Regeleinrichtung enthalten, welche die Temperatur auf einem konstanten Wert hält. Hierzu kann die Heizeinrichtung 6 Leitungsverbindungen 62 und 63 aufweisen, welche mit den jeweils zu beheizenden Komponenten verbunden sind. Die Zufuhrleitung 51 und/oder der erste akustische Resonator 10 und/oder der zweite akustische Resonator 20 können optional in Kontakt mit einem Temperatursensor stehen, welcher den IST-Wert der Temperatur erfasst und an die Heizeinrichtung 6 übermittelt. Optional kann die Heizeinrichtung 6 eine weitere Leitung 61 aufweisen, welche zur Übertragung von Soll- oder Ist-Werten an die elektronische Datenverarbeitungsanlage 93 eingerichtet sein kann.

Wie aus Figur 2 weiterhin ersichtlich ist, weist die erste photoakustische Messeinrichtung 1 im dargestellten Ausführungsbeispiel zwei erste Lichtquellen 12 auf, welche jeweils Licht unterschiedlicher Wellenlängen emittieren. Beispielsweise kann eine Lichtquelle Licht mit einer Wellenlänge von 275 nm emittieren und die andere Lichtquelle kann Licht mit 295 nm emittieren. Damit kann die erste photoakustische Messeinrichtung Aceton und Propofol nachweisen.

Die Absorption des Lichtes führt zu einer Druckschwankung im akustischen Resonator, welcher als Schallsignal mit zumindest einem ersten Mikrofon 11 nachweisbar ist. Das Mikrofon 11 kann beispielsweise ein MEMS-Mikrofon sein.

Die zweite photoakustische Messeinrichtung 2 weist im dargestellten Ausführungsbeispiel zwei zweite Lichtquellen 22 auf. Dabei ist eine Lichtquelle dazu eingerichtet und bestimmt, Licht mit einer Wellenlänge von 295 nm zu emittieren. Die andere Lichtquelle ist dazu eingerichtet und bestimmt, Licht mit einer Wellenlänge von 308 nm zu emittieren. Damit ist die zweite photoakustische Messeinrichtung 2 sensitiv auf Aceton. Die Konzentration von Propofol kann dann aus der Differenz der Messignale der ersten photoakustischen Messeinrichtung 1 und der zweiten photoakustischen Messeinrichtung 2 bestimmt werden.

Auch das Licht der zumindest einen zweiten Lichtquelle 22 führt zu einem photoakustischen Signal, welches mit einem zweiten Mikrofon 21 nachweisbar ist. Auch das zweite Mikrofon 21 kann ein MEMS-Mikrofon sein. Die Signale der ersten und zweiten Mikrofone 11, 21 können einem Messverstärker 7 über dessen Eingänge 71 zugeführt werden. Der Messverstärker 7 kann die Signale analog verstärken und über seine Ausgänge 72 ausgeben. Die Ausgänge 72 können mit dem A/D-Wandler 92 verbunden sein und die digitalisierten Messwerte der elektronischen Datenverarbeitungsanlage 93 zuführen.

Die ersten und zweiten Lichtquellen 12, 22 werden über eine Einrichtung 4 zur Modulation mit der Resonanzfrequenz einer Resonatormode betrieben. Durch die resonante Anregung können die akustischen Resonatoren das photoakustische Signal verstärken, sodass dieses mit verbessertem Signal-RauschVerhältnis durch die ersten und zweiten Mikrofone 11, 21 nachgewiesen werden kann.

Da sich die Frequenz einer zur Messung verwendeten Resonatormode der akustischen Resonatoren 10, 20 mit der Temperatur und der Gaszusammensetzung ändern, weist die Vorrichtung 9 weiterhin eine Einrichtung 3 zur Erfassung einer Resonanzfrequenz einer Resonatormode auf. Die auf diese Weise erfasste Frequenz wird über einen ersten Ausgang 31 der Einrichtung zur Datenverarbeitung 93 zur Verfügung gestellt. Darüber hinaus ist der erste Eingang 31 mit dem Eingang 41 der Einrichtung 4 zur Modulation der Lichtquellen verbunden.

Die Einrichtung 3 zur Erfassung einer Resonanzfrequenz kann optional einen zweiten Ausgang 32 aufweisen, welcher ein Clock-Signal für den A/D-Wandler 92 bereit stellt. Die Frequenz am zweiten Ausgang 32 kann ein Vielfaches der Frequenz am Ausgang 31 sein, sodass die Arbeitsfrequenz des A/D-Wandlers ein Vielfaches der Modulationsfrequenz der ersten und zweiten Lichtquellen beträgt. Hierdurch kann die Messgenauigkeit erhöht sein. Der Multiplikator der Frequenzen kann in einigen Ausführungsformen der Erfindung 2ⁿ sein. In einigen Ausführungsbeispielen kann 1 ≤ n ≤ 8 gewählt sein.

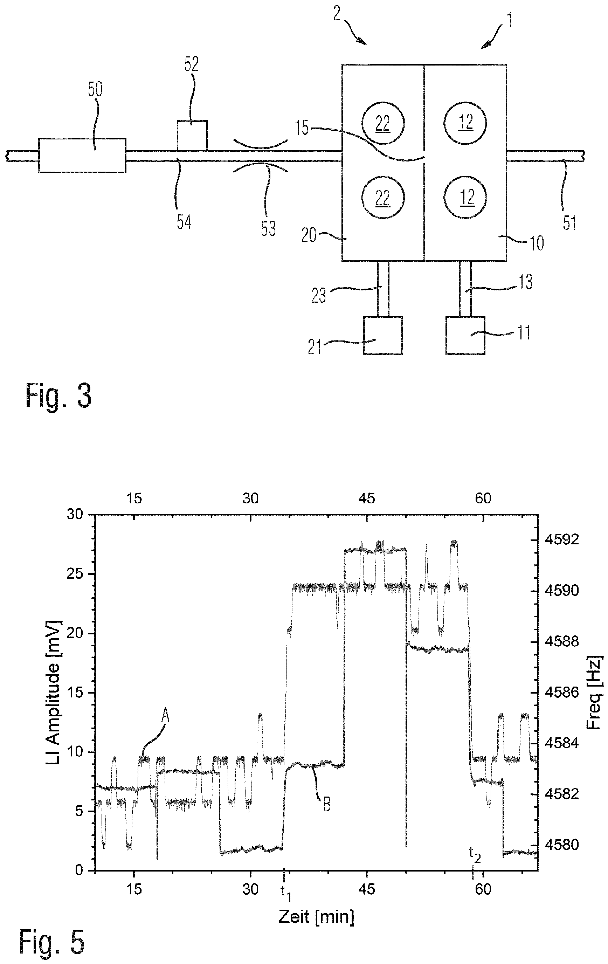

Die akustischen Resonatoren, die Lichtquellen und die Mikrofone sind anhand der Figur 3 nochmals vergrößert dargestellt. Darüber hinaus zeigt Figur 3 einen anderen Schnitt durch die Resonatoren 10 und 20. Die Schnittführung ist in etwa orthogonal zur vorstehenden Figur 2 gewählt. Gleiche Bestandteile der Erfindung sind mit gleichen Bezugszeichen versehen, sodass sich die nachfolgende Beschreibung auf die wesentlichen Unterschiede beschränkt.

Wie aus Figur 3 ersichtlich ist, sind die ersten und zweiten Mikrofone 11, 21 über erste und zweite Verbindungsrohre 13, 23 mit dem jeweiligen akustischen Resonator 10, 20 verbunden. Dieses Merkmal hat die Wirkung, dass die ersten und zweiten Mikrofone 11, 21 auf niedrigerer Temperatur gehalten werden können als die jeweils zugeordneten akustischen Resonatoren 10, 20. Hierdurch kann eine vorzeitige Alterung oder Beschädigung der Mikrofone vermieden werden.

Figur 3 zeigt weiterhin, dass in der Abfuhrleitung 54 ein Drosselventil 53 und eine Druckmesseinrichtung 52 vorhanden ist. Das Drosselventil 53 kann einen Druckverlust von etwa 20 hPa bis etwa 40 hPa bewirken. Der Drucksensor 52 kann einen Messbereich von weniger als 100 hPa aufweisen. In einigen Ausführungsformen der Erfindung kann der Drucksensor 52 dazu eingerichtet sein, einen Druckverlust über das Drosselventil 53 zu erfassen. Aus dem Druckverlust kann somit der Durchfluss durch die akustischen Resonatoren mit hoher Genauigkeit erfasst werden. In anderen Ausführungsformen der Erfindung können das Drosselventil 53 und der Drucksensor 52 auch in der Zufuhrleitung 51 angeordnet sein statt in der Abfuhrleitung 54.

Weiterhin zeigt Fig. 3, dass die akustischen Resonatoren 10, 20 eine gemeinsame Trennwand aufweisen, welche eine Bohrung 15 aufweist, welche ein Überströmen des zugeführten Gases vom ersten Resonator 10 in den zweiten Resonator 20 ermöglicht. Die Bohrung 15 kann in einigen Ausführungsformen der Erfindung einen Durchmesser von etwa 0,1 mm bis etwa 1 mm oder von etwa 0,5 mm bis etwa 2 mm aufweisen.

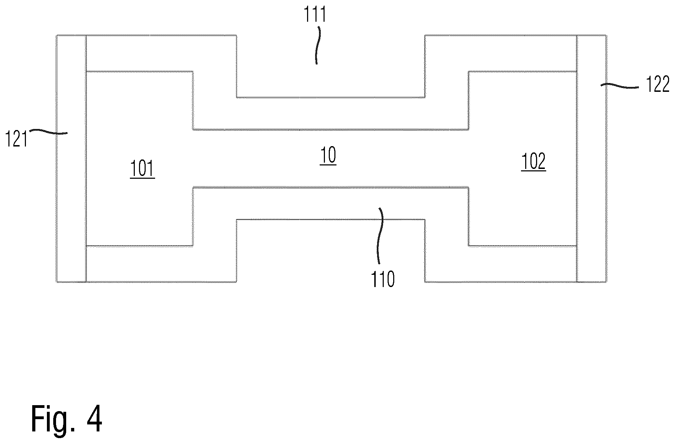

Figur 4 zeigt eine Ausführungsform eines photoakustischen Resonators 10 im Schnitt. In der in Fig. 4 dargestellten Ausführungsform ist der photoakustische Resonator 10 durch eine Wandung 110 begrenzt. Die Wandung kann ein Metall oder eine Legierung oder einen Kunststoff oder Glas enthalten oder daraus bestehen. Die Innenseite kann mit einer optionalen Beschichtung versehen sein.

Die gegenüberliegenden Seitenflächen des photoakustischen Resonators 10 sind offen, d.h. im Wesentlichen materialfrei ausgeführt und grenzen an zwei Hohlkörper 101 und 102 an. Durch die offenen Seitenflächen besteht die Möglichkeit eines Gasaustausches zwischen den Hohlkörpern 101 und 102 und dem photoakustischen Resonator 10. Die Hohlkörper stellen jeweils ein Puffervolumen dar. Dieses kann in einzelnen Ausführungsformen der Erfindung folgende Wirkungen haben, wobei nicht alle Vorteile in allen Ausführungsformen gleichermaßen realisiert sein müssen:
- Die Hohlkörper 101, 102 sorgen dafür, dass die offenen Seitenflächen des Resonators den photoakustisch erzeugten Schalldruck reflektieren. In einigen Ausführungsformen können mehr als 90 % des photoakustisch erzeugten Schalldruck reflektiert werden.
- In den Hohlkörpern 101, 102 können die Zu- und Abfuhrleitungen 51 und 54 münden. Auch die vorstehend beschriebene Bohrung 15, welche ein Überströmen des zugeführten Gases vom ersten Resonator 10 in den zweiten Resonator 20 ermöglicht, kann in angrenzenden Hohlkörpern 101, 102 angebracht sein. Hierdurch wird eine Störung des photoakustischen Signals durch Strömungsgeräusche verhindert oder reduziert.
- Der oder die Hohlkörper 101, 102 können Teil eines freilaufenden akustischen Oszillators sein, welcher die Erfassung der Frequenz zumindest einer Eigenmode des akustischen Resonators ermöglicht, so dass die Modulationsfrequenz der Lichtquelle bestimmt werden kann.

Da die Hohlkörper 101, 102 einen größeren Querschnitt aufweisen als der photoakustische Resonator 10, bilden sich Ausnehmungen 111, in welche ein nicht dargesteltles Mikrofon positioniert werden kann. Die dem photoakustischen Resonator 10 abgewandten Seiten der Hohlkörper 101, 102 weisen jeweils ein Fenster 121, 122 auf, durch welche das Licht der Lichtquellen 12, 22 in den photoakustischen Resonator 10 eingekoppelt werden kann. Der zweite photoakustische Resonator 20 kann einen identischen Aufbau aufweisen. Somit kann das zu analysierende Gas durch die Zufuhrleitung 51 in den ersten Hohlkörper 101 eintreten und von dort durch den photoakustischen Resonator 10 in den zweiten Hohlkörper 102 strömen. Von dort strömt das Gas über die Bohrung 15 (in fig. 4 nicht sichtbar) in einen Hohlkörper der zweiten Messeinrichtung. Danach fließt es durch den zweiten photoakustischen Resonator 20 in den anderen Hohlkörper der zweiten Messeinrichtung und durch die Abfuhrleitung 54 zur Pumpe 50.

Die Funktionsweise der Einrichtung 3 zur Erfassung der Modulationsfrequenz wird nochmals anhand eines Ausführungsbeispiels in der Figur 5 erläutert. Dargestellt ist die Modulationsfrequenz in Kurve A auf der rechten Ordinate sowie das photoakustische Signal in Kurve B auf der linken Ordinate. Die Messwerte werden gegen die Zeit aufgetragen, wobei sich die Gaszusammensetzung innerhalb der akustischen Resonatoren über die Zeit ändert.

Im Zeitbereich von 0 bis 34 Minuten und von 58 bis 67 Minuten wird Raumluft mit einem Volumenstrom von 100 cm³/min eingesaugt. Wie die Kurve A zeigt, stellt sich hierbei eine im Wesentlichen konstante Frequenz der Resonatormode ein. Die dargestellten kleinen Sprünge zeigen die digitale Auflösung des Frequenztrackings von etwa 1,7 Hz.

Zwischen 34 und 58 Minuten (auf der Abszisse mit t₁ und t₂ bezeichnet) wird Raumluft mit 90 cm³/min und ein Gasgemisch mit 10 cm³/min angesaugt und durch den akustischen Resonator gefördert. Das Gasgemisch enthält dabei 500 ppm Aceton in Stickstoff. Wie in Figur 5 gezeigt wird, erfasst das Frequenztracking die sich um etwa 5 Hz ändernde Resonanzfrequenz der Resonatormode. Das photoakustische Signal in Kurve B kann durch die Anpassung der Modulationsfrequenz weiterhin erfasst werden.

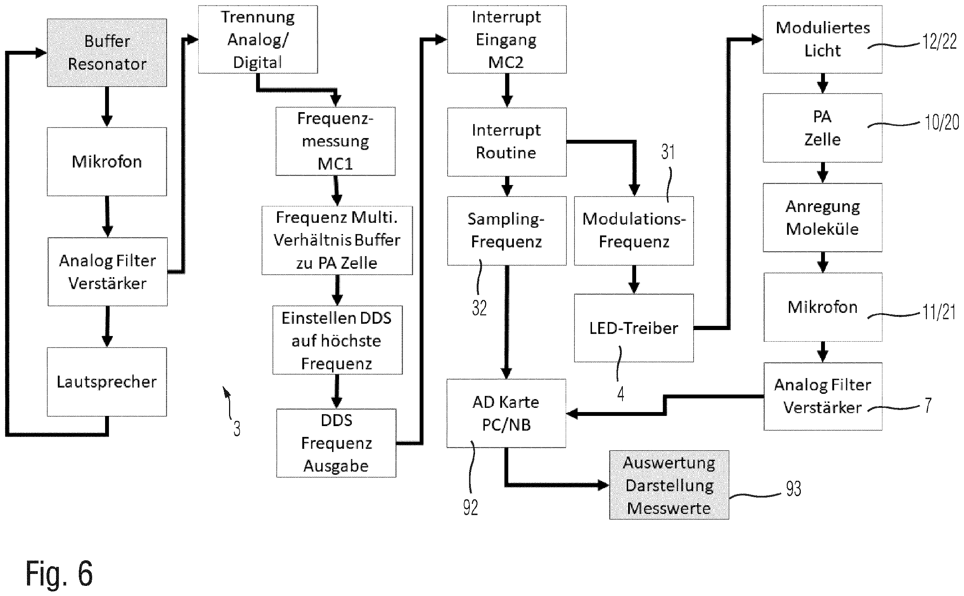

Anhand der Figur 6 wird die Funktionsweise der vorgeschlagenen Vorrichtung nochmals erläutert. Im linken Bildteil ist ein freilaufender akustischer Oszillator dargestellt, welcher aus den jeweiligen akustischen Resonatoren, einem Mikrofon, einem Analogverstärker und einem Lautsprecher besteht. Der freilaufende akustische Oszillator wird mit einer Frequenz betrieben, welche sich von der Resonanzfrequenz der ersten und zweiten akustischen Resonatoren signifikant unterscheidet. Beispielsweise kann die Frequenz etwa 2-fach bis etwa 4-fach höher sein. Die sich einstellende Frequenz des freilaufenden akustischen Oszillators ist ein Maß für die Änderung der Schallgeschwindigkeit und damit auch ein Maß für die Änderung der Frequenz der Resonatormode, welche für die photoakustische Messung verwendet wird.

Die sich einstellende Frequenz kann gemessen werden, um diese nachfolgend der Einrichtung 4 zur Modulation der Lichtquellen zuzuführen. Die modulierten Lichtquellen regen sodann das photoakustische Signal an, welches mit den ersten und zweiten Mikrofonen erfasst, verstärkt und digitalisiert wird.

Selbstverständlich ist die Erfindung nicht auf die dargestellten Ausführungsformen beschränkt. Die vorstehende Beschreibung ist daher nicht als beschränkend, sondern als erläuternd anzusehen. Die nachfolgenden Ansprüche sind so zu verstehen, dass ein genanntes Merkmal in zumindest einer Ausführungsform der Erfindung vorhanden ist. Dies schließt die Anwesenheit weiterer Merkmale nicht aus. Die nachfolgenden Ansprüche sind nicht so zu verstehen, dass ein genanntes Merkmal in jeder Ausführungsform der Erfindung vorhanden sein muss. Sofern die Ansprüche und die vorstehende Beschreibung "erste" und "zweite" Ausführungsformen definieren, so dient diese Bezeichnung der Unterscheidung zweier gleichartiger Ausführungsformen, ohne eine Rangfolge festzulegen.

## Patentansprüche

1. Vorrichtung (9) zur Erfassung von zumindest zwei Spurengasen in einer Ausatemluft (55), enthaltend
eine erste photoakustische Messeinrichtung (1) mit einem ersten akustischen Resonator (10), zumindest einem ersten Mikrofon (11) und zumindest einer ersten Lichtquelle (12),
eine Einrichtung (3) zur Erfassung einer Resonanzfrequenz einer Resonatormode des ersten akustischen Resonators (10),
eine Einrichtung (4) zur Modulation der ersten Lichtquelle (12) mit der Resonanzfrequenz der Resonatormode,
**dadurch gekennzeichnet, dass**
die Vorrichtung (9) weiterhin eine zweite photoakustische Messeinrichtung (2) enthält, mit einem zweiten akustischen Resonator (20), zumindest einem zweiten Mikrofon (21) und zumindest einer zweiten Lichtquelle (22),
wobei die Einrichtung (4) zur Modulation der ersten Lichtquelle (12) dazu eingerichtet ist, auch die zweite Lichtquelle (22) mit der Resonanzfrequenz der Resonatormode des ersten akustischen Resonators (10) zu modulieren oder
eine weitere Einrichtung (3) zur Erfassung einer Resonanzfrequenz einer Resonatormode des zweiten akustischen Resonators (20) und eine weitere Einrichtung (4) zur Modulation der zweiten Lichtquelle (12) mit der Resonanzfrequenz der Resonatormode des zweiten akustischen Resonators (20) vorhanden sind.

2. Vorrichtung nach Anspruch 1, **dadurch gekennzeichnet, dass** der erste akustische Resonator (10) und der zweite akustische Resonator (20) in Strömungsrichtung der Ausatemluft (55) hintereinander angeordnet sind.

3. Vorrichtung nach Anspruch 1 oder 2, **dadurch gekennzeichnet, dass** die erste Lichtquelle (12) dazu eingerichtet ist, Licht mit einer Wellenlänge von etwa 270 nm bis etwa 300 nm zu emittieren und/oder
dass die erste Lichtquelle (12) dazu eingerichtet ist, Licht mit einer Wellenlänge von etwa 270 nm bis etwa 280 nm zu emittieren und/oder
dass die zweite Lichtquelle dazu (22) eingerichtet ist, Licht mit einer Wellenlänge von etwa 290 nm bis etwa 310 nm zu emittieren.

4. Vorrichtung nach einem der Ansprüche 1 bis 3, **dadurch gekennzeichnet, dass** die erste photoakustische Messeinrichtung (1) und/oder die zweite photoakustische Messeinrichtung (2) jeweils eine Mehrzahl erster und zweiter Lichtquellen (12, 22) aufweisen und/oder
dass die ersten Lichtquellen (12) dazu eingerichtet ist, Licht mit 275 nm und 295 nm zu emittieren und/oder
dass die zweiten Lichtquellen (22) dazu eingerichtet ist, Licht mit 295 nm und 308 nm zu emittieren.

5. Vorrichtung nach einem der Ansprüche 1 bis 4, weiterhin enthaltend zumindest eine Förderpumpe (50), welche dazu eingerichtet ist, die Ausatemluft über eine Zufuhrleitung (51) durch den ersten und zweiten akustischen Resonator (10, 20) zu fördern.

6. Vorrichtung nach Anspruch 5, weiterhin enthaltend eine Druckmesseinrichtung (52) und ein Drosselventil (53) in der Abfuhrleitung (51).

7. Vorrichtung nach einem der Ansprüche 1 bis 6, weiterhin enthaltend zumindest eine Heizeinrichtung (6), mit welcher der erste akustische Resonator (10) und/oder der zweite akustische Resonator (20) und/oder die Zufuhrleitung (51) auf eine Temperatur von etwa 80°C bis etwa 180°C oder von etwa 100°C bis etwa 120°C bringbar sind.

8. Vorrichtung nach einem der Ansprüche 1 bis 7, **dadurch gekennzeichnet, dass** zumindest eine Teilfläche der Innenflächen des ersten akustischen Resonators (10) und/oder des zweiten akustischen Resonators (20) und/oder der Zufuhrleitung (51) mit einer Beschichtung versehen sind oder
dass zumindest eine Teilfläche der Innenflächen des ersten akustischen Resonators (10) und/oder des zweiten akustischen Resonators (20) und/oder der Zufuhrleitung (51) mit einer Beschichtung versehen sind, welche Silicium und/oder Siliciumoxid enthält oder daraus besteht.

9. Vorrichtung nach einem der Ansprüche 1 bis 8, **dadurch gekennzeichnet, dass** zwischen dem ersten Mikrofon (11) und dem ersten akustischen Resonator (10) ein erstes Verbindungsrohr (13) angeordnet ist und/oder
dass zwischen dem zweiten Mikrofon (21) und dem zweiten akustischen Resonator (20) ein zweites Verbindungsrohr (23) angeordnet ist.

10. Vorrichtung nach Anspruch 9, **dadurch gekennzeichnet, dass** das erste und/oder zweite Verbindungsrohr (13, 23) Polytetrafluorethylen enthält oder daraus besteht.

11. Verfahren zur Bestimmung von zumindest zwei Spurengasen in einer Ausatemluft mit folgenden Schritten
Zuführen eines Teilstromes der Ausatemluft zu einer ersten photoakustischen Messeinrichtung (1) mit einem ersten akustischen Resonator (10), zumindest einem ersten Mikrofon (11) und zumindest einer ersten Lichtquelle (12),
Erfassen einer Resonanzfrequenz einer Resonatormode des ersten akustischen Resonators (10),
Modulieren der ersten Lichtquelle (12) mit der Resonanzfrequenz der Resonatormode des ersten akustischen Resonators (10),
Erfassen des photoakustischen Signals im ersten akustischen Resonator (10) mit dem zumindest einen ersten Mikrofon (11),
**dadurch gekennzeichnet, dass**
der Teilstrom der Ausatemluft weiterhin einer zweiten photoakustischen Messeinrichtung (2) zugeführt wird, welche einen zweiten akustischen Resonator (20), zumindest ein zweites Mikrofon (21) und zumindest eine zweite Lichtquelle (22) enthält,
wobei auch die zweite Lichtquelle (22) mit der Resonanzfrequenz der Resonatormode des ersten akustischen Resonators (10) moduliert wird oder
eine Resonanzfrequenz einer Resonatormode des zweiten akustischen Resonators (20) erfasst wird und die zweite Lichtquelle (22) mit der Resonanzfrequenz der Resonatormode des zweiten akustischen Resonators (20) moduliert wird, und
Erfassen des photoakustischen Signals im zweiten akustischen Resonator (20) mit dem zumindest einen zweiten Mikrofon (21).

12. Verfahren nach Anspruch 11, **dadurch gekennzeichnet, dass** der erste akustische Resonator (10) und der zweite akustische Resonator (20) von der Ausatemluft (55) sequenziell durchströmt werden.

13. Verfahren nach einem der Ansprüche 11 bis 12, **dadurch gekennzeichnet, dass** die erste photoakustische Messeinrichtung (1) zwei erste Lichtquellen (12) aufweist, welche Licht mit 275 nm und 295 nm emittieren und/oder dass die zweite photoakustische Messeinrichtung (2) zwei zweite Lichtquellen (22) aufweist, welche Licht mit 295 nm und 308 nm emittieren.

14. Verfahren nach einem der Ansprüche 11 bis 13, **dadurch gekennzeichnet, dass** die Ausatemluft mit einer Förderpumpe (50) über eine Zufuhrleitung (51) durch den ersten und zweiten akustischen Resonator (10, 20) gefördert wird, wobei in der Zufuhrleitung (51) eine Druckmesseinrichtung (52) und ein Drosselventil (53) angeodnet sind und aus dem Druckverlust über das Drosselventil (53) der Durchfluss berechnet wird.

15. Verfahren nach einem der Ansprüche 11 bis 14, **dadurch gekennzeichnet, dass** die zwei Spurengase ausgewählt sind aus Propofol und Aceton.
